(19) Europäisches Patentamt
European Patent Office
Office européen des brevets

(11) **EP 4 154 035 B1**

(12) **FASCICULE DE BREVET EUROPEEN**

(45) Date de publication et mention de la délivrance du brevet:
**21.02.2024 Bulletin 2024/08**

(21) Numéro de dépôt: **21721152.3**

(22) Date de dépôt: **31.03.2021**

(51) Classification Internationale des Brevets (IPC):
**G01T 1/02** *(2006.01)*    **A61B 6/00** *(2024.01)*
**G01T 1/20** *(2006.01)*    **G01T 1/29** *(2006.01)*

(52) Classification Coopérative des Brevets (CPC):
**G01T 1/201; A61B 6/5205; G01T 1/023;**
**G01T 1/2985;** A61B 6/4441

(86) Numéro de dépôt international:
**PCT/FR2021/050562**

(87) Numéro de publication internationale:
**WO 2021/234234 (25.11.2021 Gazette 2021/47)**

(54) **PROCEDE DE TRAITEMENT DES DONNEES RELATIVES A UN EXAMEN RADIOLOGIQUE D'UN PATIENT**

VERFAHREN ZUR VERARBEITUNG VON DATEN EINER RADIOLOGISCHEN UNTERSUCHUNG EINES PATIENTEN

METHOD FOR PROCESSING DATA RELATING TO A RADIOLOGICAL EXAMINATION OF A PATIENT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **21.05.2020 FR 2005393**

(43) Date de publication de la demande:
**29.03.2023 Bulletin 2023/13**

(73) Titulaires:
 • **Fibermetrix**
 **67960 Entzheim (FR)**
 • **Alara Expertise**
 **67960 Entzheim (FR)**

(72) Inventeurs:
 • **MUNIER, Mélodie**
 **67540 OSTWALD (FR)**
 • **CARBILLET, Fanny**
 **67540 OSTWALD (FR)**
 • **PRUDHOMME, Pierre-Benoît**
 **STRASBOURG 67000 (FR)**
 • **GUILLOCHON, Nicolas**
 **90000 BELFORT (FR)**

(74) Mandataire: **IP Trust**
 **2, rue de Clichy**
 **75009 Paris (FR)**

(56) Documents cités:
 **WO-A1-2019/064652**    **US-A1- 2006 018 435**
 **US-A1- 2006 027 756**

**Description**

Domaine de l'invention

La présente invention se rapporte au domaine de la tomodensitométrie et plus particulièrement le domaine de la caractérisation des doses de rayonnement reçus par les patients lors d'examens d'imagerie CT (du terme anglais « computed tomography »). Les scanners actuels, mesurent l'intensité des rayons délivrés par le tube à rayons X après qu'ils ont été partiellement absorbés durant leur passage à travers le corps, à l'aide d'un cadre tournant portant une source entraînée en rotation autour du patient, ou à l'aide d'un cadre portant des récepteurs immobiles formant un anneau complet.

Les données obtenues sont ensuite traitées par ordinateur, ce qui permet de recomposer des vues en coupes bidimensionnelles puis des vues en trois dimensions des organes. On peut faire ressortir le contraste de certains tissus, en particulier des vaisseaux sanguins, en injectant un produit dit « de contraste » (un complexe de l'iode) qui a la propriété d'absorber fortement les rayons X et donc de rendre très visibles les tissus où ce produit est présent (qui apparaissent alors hyperdenses, c'est-à-dire plus « blancs » sur l'image). Grâce aux tomodensitomètres multidétecteurs (ou multi-barrettes) à acquisition spiralée (déplacement lent de la table d'examen durant l'acquisition), on obtient depuis les années 1990 une exploration très précise d'un large volume du corps humain pour un temps d'acquisition de quelques secondes.

Un scanner CT peut produire plus de 1 000 images de la partie du corps d'un patient à scanner, généralement la tête, le thorax, l'abdomen ou le bassin. Comme pour toute radiographie, l'exposition répétée aux rayons X peut être nocive pour l'organisme, mais le rapport bénéfice/risque lié à l'irradiation penche largement en faveur de la tomodensitométrie, lorsque la demande d'examen est justifiée, ce qui en fait une technique d'imagerie médicale de plus en plus pratiquée. L'imagerie par résonance magnétique (IRM), technique non irradiante utilisant les champs magnétiques, représente une solution de remplacement ou un complément pour certaines applications mais ne peut pas remplacer l'imagerie scanographique dans toutes les indications.

La tomodensitométrie est devenue, en raison de l'intérêt médical de ce type d'examen, le plus grand contributeur à la dose de rayonnement dans la population. La dose de rayonnement associée à la tomodensitométrie a toujours été préoccupante, mais l'utilisation accrue de la tomodensitométrie dans un large éventail de situations diagnostiques couplée aux capacités de sortie élevées des tomodensitomètres modernes accentue ces préoccupations. Il devient nécessaire de connaître avec précision la quantité de rayonnement réellement émise et donc la dose réellement reçue par le patient et évaluer l'intérêt de la procédure radiologique, c'est-à-dire la justifier l'acte en regard des techniques alternatives, donnant lieu à une irradiation moindre, voire nulle et, le cas échéant tout mettre en oeuvre afin de minimiser et rationaliser la dose délivrée au patient.

Etat de la technique

Afin de fournir à l'opérateur des informations pertinentes concernant la dose de rayonnement à laquelle un patient a été soumis pendant une tomodensitométrie (TDM), on a proposé dans le brevet américain US7627079B2 un procédé consistant à :

- Soumettre un patient à un faisceau de rayons X provenant d'une source de rayons X;
- Déterminer une dose de rayonnement de rayons X primaire à partir du faisceau de rayons X;
- Déterminer une dose de rayonnement X diffusé à partir du faisceau de rayons X.

La détermination de la dose de rayonnement radiologique primaire comprend le calcul d'une distribution de dose primaire à partir des données d'image de scanographie générées pendant le scan du patient et le calcul d'une dose de rayonnement délivrée au patient sur la base de la dose de rayonnement X primaire et de la dose de rayonnement X diffusé.

Les propriétés utilisées pour calculer la distribution de dose primaire comprennent un schéma de modulation mA de la source de rayons X utilisé pour calculer la dose de rayonnement délivrée au patient.

Ce procédé de l'art antérieur utilise comme information d'entrée l'ensemble de données d'image de tomographie numérique généré pendant le balayage du patient et un ou plusieurs paramètres relatifs à une source de rayons X sont utilisés pour calculer la dose de rayonnement délivrée au patient en fonction de l'ensemble de données d'image et du ou des paramètres de la source de rayons X.

La dose de rayonnement est trouvée en calculant une distribution de dose de rayons X primaire et une distribution de dose de rayons X dispersés à partir du jeu de données d'images CT et en prenant la somme de la distribution de dose de rayons X primaire et de la distribution de dose de rayons X diffusée.

Cette solution n'est pas vraiment satisfaisante car elle fournit un indicateur d'exposition déterminé à partir d'un modèle théorique plus ou moins pertinent et non pas de la réalité du rayonnement auquel le patient a été soumis.

La demande de brevet coréenne KR20150061520A décrit un autre exemple de procédé d'évaluation de la dose de rayonnement afin qu'un utilisateur puisse facilement calculer quantité de rayonnement reçue par le patient lors d'une séance d'imagerie dans un scanner tomographique. Cette solution est également basée sur une estimation de la dose à laquelle est exposée le patient.

On connaît aussi le brevet américain US7627079B2 qui concerne le calcul de la dose de rayonnement délivrée à un patient lors d'une tomographie par ordinateur. L'ensemble des données d'image de la tomographie par ordinateur généré lors du balayage du patient et un ou plusieurs paramètres concernant une source de rayons X sont utilisés pour calculer la dose de rayonnement fournie au patient en tant que fonction dudit ensemble de données et du ou des paramètres supplémentaires de ladite source. La dose de rayonnement est généralement trouvée en calculant une répartition d'une dose de rayons X principaux et une répartition d'une dose de rayons X dispersés à partir dudit ensemble de données et en prenant la somme desdites répartitions.

Il s'agit également dans cette solution d'une estimation indirecte de la dose délivrée au patient.

Le brevet US8189740 décrit un appareil de tomodensitométrie à rayons X, un appareil d'assistance au plan de balayage et un procédé d'assistance au plan de balayage qui améliore la sensibilisation de l'opérateur à la dose d'exposition aux radiations.

L' appareil CT à rayons X qui transmet des rayons X vers un sujet sur la base d'une condition de balayage définie et reconstruit des images à partir de rayons X détectés qui traversent le sujet, y compris un unité de définition de plan de balayage configurée pour définir la condition de balayage, une unité de stockage de dose de référence configurée pour stocker des informations de dose de référence comprenant une relation entre des informations d'attribut sur une pluralité de types de sujets et la dose de référence correspondante et une unité de calcul de dose d'exposition configurée pour calculer une exposition dose correspondant à la condition de balayage définie conformément au moment du réglage de la condition de balayage.

On connaît encore le brevet FR3053799 de la demanderesse, décrivant un dispositif de détermination en temps réel de la dose déposée lors d'un examen radiologique de manière simple et précise. Ce brevet décrit un dispositif de détermination d'une dose déposée, comprenant: une sonde de mesure, comprenant une sonde optique en U définissant deux extrémités de sortie, ladite sonde optique comprenant au moins une portion active en matériau scintillant et destinée à émettre des photons de scintillation sous l'effet d'un rayonnement ionisant incident et au moins deux portions de transport, disposées de part et d'autre de la portion active et configurées pour porter les photons de scintillation émis par la portion active vers les deux sorties; un système de détection comprenant au moins deux photodétecteurs, chaque photodétecteur étant connecté à une extrémité de sortie respective de la sonde optique en U afin de recevoir et de compter les photons de scintillation reçus de ladite extrémité de sortie; et un module de traitement, configuré pour déterminer la dose déposée à partir des mesures effectuées par lesdits photodétecteurs.

Cette solution est plus efficace que les solutions exploitant uniquement un modèle théorique et les images de scan CT, et permet de fournir des informations réelles sous la forme d'indicateurs correspondant à deux grandeurs dosimétriques spécifiques, à savoir l'indice de dose du scanner (IDSV ou CTDI) et la longueur de dose du produit (PDL ou DLP pour le produit de longueur de dose).

La demande de brevet WO2012129661A1 propose un dosimètre plan et volumétrique à utiliser avec une machine de radiothérapie ayant une source de rayonnement. Le dosimètre comprend un ensemble scintillant comprenant une pluralité de fibres optiques scintillantes et configuré pour générer un flux lumineux en réponse à la distribution de dose incidente sur celui-ci à partir de la source de rayonnement, et un photodétecteur utilisable pour convertir l'énergie optique émise par l'ensemble scintillant en signaux électriques pour déterminer la distribution de dose réelle bidimensionnelle (2D) ou tridimensionnelle (3D) incidente sur l'ensemble scintillant en utilisant un algorithme de reconstruction tomographique.

Le brevet US8714818 décrit un autre exemple de système d'imagerie médicale comprenant une source de rayonnement, un capteur de rayonnement, une unité de collecte de données et un système d'imagerie. La source de rayonnement a une ouverture pour diriger un faisceau de rayonnement collimaté dans une direction vers un patient. Le capteur de rayonnement est disposé à proximité de l'ouverture et à l'intérieur du faisceau de rayonnement collimaté pour mesurer une fluence du faisceau de rayonnement collimaté. L'unité de collecte de données est disposée pour collecter le rayonnement du faisceau collimaté après interaction avec le patient. Le système d'imagerie est en communication avec l'unité de collecte de données et configuré pour générer une image d'une partie du patient à partir du rayonnement collecté.

On connaît aussi la demande WO2019/064652 qui concerne un poste de travail qui joint à une image radiologique conforme à la norme Dicom des données de dose d'exposition créées par un système de mesure d'exposition à l'aide d'informations concernant la date et l'heure de capture d'image de l'image radiologique conforme à la norme Dicom et d'informations concernant la date et l'heure de capture d'image dans les données de dose d'exposition créées par le système de mesure d'exposition. Le poste de travail calcule ainsi la dose d'exposition de chaque image radiologique. En outre, le poste de travail calcule la dose d'exposition pour chaque examen radiologique en fonction de données concernant l'heure de début d'examen et l'heure de fin reçus en provenance d'une unité de console.

La demande US2006/018435 concerne un ensemble de tomodensitométrie comprenant un ensemble portique à rayons X, une source de rayons X projetant un faisceau de rayons X, un ensemble détecteur positionné à l'opposé de la source de rayons X et recevant le faisceau de rayons X, et un mécanisme de commande en communication avec la source de rayons X et l'ensemble détecteur. Le mécanisme de commande comprend une logique adaptée pour : exécuter au moins un balayage de reconnaissance de l'objet pour produire une première image de balayage de reconnaissance ; générer un modèle de patient elliptique sur la base de la première image de balayage; faire correspondre le modèle de patient elliptique à une approximation du diamètre du fantôme ; générer un rapport de dose basé sur l'approximation du diamètre du fantôme ; et afficher ledit rapport de dose sur un affichage en communication avec le mécanisme de commande.

La demande US2006/027756 concerne un dosimètre pour mesurer un rayonnement ionisant comprenant une pluralité de capteurs de rayonnement à transistor à effet de champ à grille isolée (IGFET) espacés à des intervalles prédéterminés sur un support et des moyens pour coupler les capteurs à des moyens pour lire sélectivement les capteurs.

Inconvénients de l'art antérieur

Les solutions de l'art antérieur ne sont pas totalement satisfaisantes car elles ne permettent pas de construire d'indicateurs réellement fiables avec une définition suffisante pour déterminer la dose par zone corporelle. Or la connaissance exacte de la dose effective et pas seulement estimée par une simulation, délivré au niveau du pelvis, du thorax, du poumon, du crâne, etc... est une donnée importante pour ajuster au mieux le fonctionnement d'un scanner, et pour construire des indicateurs correspondant à des références par type de patient, par opérateur ou par équipement, et pour déterminer l'évolution temporelle des pratiques d'imagerie, ainsi que pour procéder à des comparaisons entre les doses délivrées pendant une séquence, et les doses de référence préconisées pour une séquence de scanner.

Solution apportée par l'invention

Afin de remédier à ces inconvénients, la présente invention concerne selon son acception la plus générale un procédé de traitement des données relatives à un examen radiologique d'un patient, selon la revendication 1, au moyen d'un dispositif de détermination comportant l'acquisition des doses mesurées ($C_i$, $t_i$) à une pluralité d'instant $t_i$ et l'enregistrement de ces mesures de doses d'irradiation horodatées, et l'acquisition d'un fichier numérique DICOM caractérisé en ce qu'il comporte les étapes suivantes :

- Acquisition et enregistrement du fichier numérique DICOM délivrés par le tomographe (3) pendant la réalisation d'une tomographie
- Acquisition et enregistrement des mesures horodatées des doses détectées par une fibre scintillante (5) placée sur la table (2), et des déplacements horodatés de ladite table (2)
- Interpolation desdites mesures ($C_i$, $t_i$) des données de l'image (DICOM) dans un espace interpolé commun et construction d'une table ($C_k$, $DICOM_k$) dans ledit espace interpolé
- Détermination d'une table des niveaux de doses moyennes $T_z$ dans chaque tranche T en fonction des données ($DICOM_k$, $C_k$).

De préférence le procédé comporte en outre une étape de détermination d'un facteur correctif pour la compensation de l'uniformité de mesure.

Avantageusement le procédé comporte en outre une étape de détermination d'un facteur correctif pour la compensation des effets de dépendance en énergie.

De préférence ledit facteur correctif est déterminé en fonction de la différence entre le faisceau clinique et le faisceau utilisé pour l'étalonnage du dosimètre.

Selon une variante, ledit facteur correctif est déterminé par un traitement consistant à définir dans un premier temps l'écart entre l'énergie moyenne du faisceau de rayons X clinique et l'énergie moyenne du faisceau de référence utilisé lors de l'étalonnage par une mesure de la couche de demi-atténuation (CDA) du faisceau clinique puis d'appliquer la correction du facteur d'étalonnage correspondant.

Description détaillée d'un exemple non limitatif de réalisation de l'invention

La présente invention sera mieux comprise à la lecture de la description qui suit, concernant des exemples non limitatifs de réalisation illustrés par les dessins annexés où :

- [Fig.1] la figure 1 représente une vue schématique du modèle de traitement des données selon l'invention

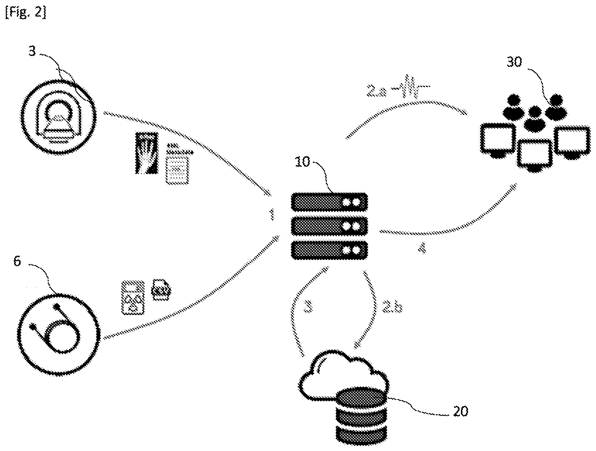

- [Fig. 2] la figure 2 représente le schéma fonctionnel du procédé objet de l'invention

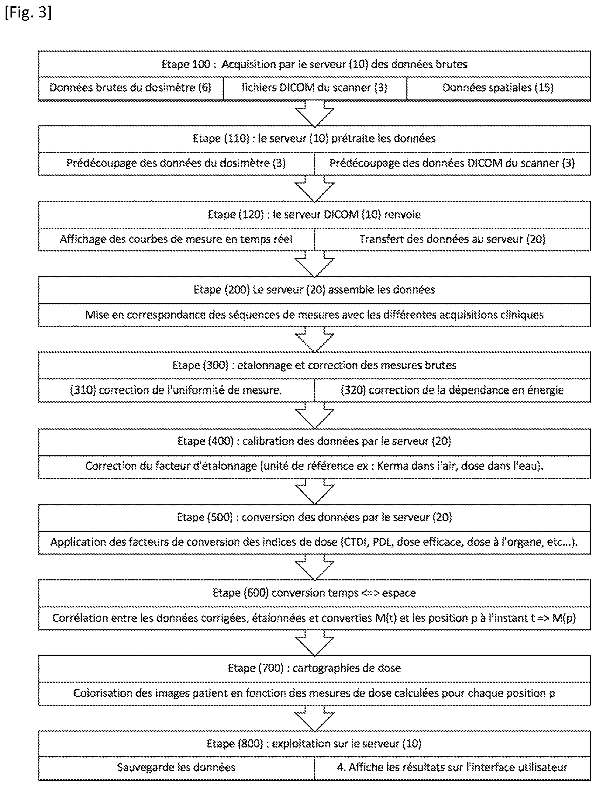

- [Fig.3] la figure 3 représente la succession des traitements objet de l'invention.

## Contexte de l'invention

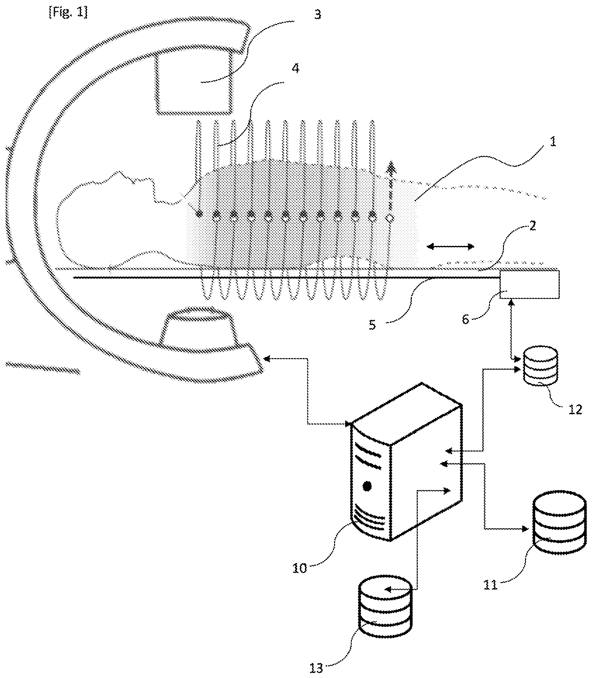

La figure 1 représente une vue schématique du fonctionnement d'un scanner tomographique numérique. Un cadre (3) rotatif supportant une source de rayon X ainsi que le détecteur ou une matrice de détecteurs se déplace selon un mouvement rotatif autour du patient (1). Ce dernier est immobilisé sur une table (2) se déplaçant selon un axe Z parallèle à l'axe de rotation du cadre rotatif. La combinaison de la translation de la table (2) et de la rotation du cadre (3) aboutit à une acquisition des données selon un mouvement relatif hélicoïdal (4). Une fibre scintillante (5), ou une série de fibre scintillante, et solidaire de la table (2) et est placée sous le patient. Cette fibre (5) est reliée optiquement à un détecteur photonique (6) intégré à un boîtier fixé sur la table (2). Ce boîtier peut comporter en outre un accéléromètre pour fournir un signal électrique intégrable pour déterminer le déplacement et donc la position de la table (2). La fibre (5) fournit une information sur la dose réelle absorbée par la fibre, présentant une densité proche de celle de l'eau, afin de ne pas perturber la mesure des détecteurs du tomodensimètre.

Un calculateur (10) reçoit :

- Les données techniques du tomographe et notamment les signaux provenant des détecteurs, et généralement déjà au format DICOM, pour les enregistrer dans une base de données (11).
- Les données provenant du dosimètre (6) pour les enregistrer dans une base de données (13).
- Des informations numériques générales correspondant à l'examen médical et au patient provenant d'équipements additionnels (30). Ces équipements additionnels permettent l'affichage en temps réel des mesures de dose effectuées par le dosimètre (6), ainsi que l'affichage post examen des informations dosimétriques traitées (dose, indices de dose, cartographie, statistiques...).

Ce calculateur peut être organisé en deux serveurs (10, 20). Dans ce cas, le premier serveur traite les données locales, concernant les scanographies réalisées avec le scanographe (3) et le dosimètre (6) local, et les traitements de ces données brutes sont réalisées sur un second serveur (20) mutualisé sur plusieurs sites d'imagerie médical, disposant chacun de son propre serveur local (10).

L'ensemble des traitements des données est réalisé entre l'étape 2.b et l'étape 3 au niveau des serveurs (10 et 20).

Les données relatives à l'exposition aux radiations du patient pendant les procédures radiologiques sont stockées dans la base de données (11) et communiquées au format DICOM de définitions d'objets d'information tomographiques. Le format numérique DICOM prévoit différentes séquences numériques, qui peuvent être utilisées pour transporter des informations d'exposition :

- l'en-tête DICOM,
- l'étape de procédure exécutée par la modalité DICOM (MPPS) et
- le rapport structuré de dose de rayonnement DICOM (RDSR)
- le rapport structuré de dose de rayonnement patient (P-RDSR).

Ces trois séquences numériques contiennent des informations sur la sortie de l'équipement (3) et fournissent les informations relatives à la sortie de l'équipement ainsi que des informations sur le patient et la procédure de scannage.

## But de l'invention

Le but de la présente invention est de fournir une solution pour déterminer de la manière la plus proche de la réalité la dose réelle à laquelle un patient a été exposé, et plus précisément de permettre la détermination de la dose réelle des principales zones du corps humain, notamment les zones particulièrement sensibles à l'exposition de radiations comme le cerveau ou la zone pelvienne, en neutralisant les artefacts résultant de l'état de l'équipement et du volume et de la nature des tissus interposés entre la sonde dosimétrique et la source de rayonnement, les spécificités corporelles du patient, la typologie de la séquence de scannage et la qualité des images à produire : des facteurs tels que le manque d'uniformité parmi les machines utilisées pour effectuer les numérisations, variant non seulement entre les fabricants mais étant également des dispositifs suffisamment complexes pour avoir des variations individuelles d'uniformité, viennent altérer les mesures dosimétriques. La dose reçue par un patient dépend aussi de la façon dont le tomographe est utilisé et de la configuration des différents paramètres pour une session d'imagerie particulière. Le vieillissement de l'équipement apporte aussi des perturbations dans la qualité des données acquises : la source de radiation, par exemple le tube à rayons X, a tendance à se dégrader avec le temps lorsque la machine est utilisée. Pour obtenir une qualité d'image similaire, une machine a tendance à devoir fonctionner à un courant plus élevé (mA) à mesure que l'efficacité du tube diminue.

Il vise aussi à corriger les erreurs de mesure du dosimètre seul, recueillant une dose réelle de rayonnement pendant la séquence de scan, mais avec un modèle approximatif et des aberrations dues au positionnement de la fibre sous le patient, qui introduit un masquage partiel et non modélisable des radiations mesurées par la fibre scintillante.

Cet objectif est atteint par l'invention en combinant trois sources d'informations de natures différentes, et présentant des temporalités différentes :

- des fichiers d'irradiations issus du dosimètre (6), reçus par le calculateur (10) sous forme de signaux brut échantillonnés dans le temps : une valeur numérique de dose toute les millisecondes tout au long de l'examen
- Des images DICOM, comprenant des métadonnées (tags DICOM) provenant du scanner (3)
- Un rapport mis en forme par le serveur (10), issu du rapport de dose et/ou du DICOM SR, fournit également par le scanner (3).

L'invention porte particulièrement sur la nature des traitements appliqués à ces données et notamment à leurs combinaisons pour fournir des indicateurs fiables de doses effectives, par zone du corps du patient. Le procédé consiste à appliquer une succession d'étapes :

- Etape (100) : acquisition et enregistrement des mesures brutes
- Etape (110) : prétraitement des données
- Etape (120) : exploitation des données
- Etape (200) : mise en correspondance des données patient
- Etape (300) : correction des mesures brutes et étalonnage
- Etape (400) : correction du facteur d'étalonnage
- Etape (500) : conversion en indicateurs de dose CTDI et DLP
- Etape (600) : conversion temps-espace
- Etape (700) : cartographie des doses
- Etape (800) : exploitation des données

Etape (100) : Acquisition et enregistrement des mesures brutes

Cette étape (100) concerne l'acquisition et l'enregistrement des données de mesure provenant d'une part du dosimètre (6) et d'autre part les fichiers DICOM du scanographe (3) ainsi que les données spatiales (15). Les données spatiales peuvent être fournies par :

- Un accéléromètre ou tout autre appareil de télémétrie intégré au dosimètre (6) ET/OU
- Les fichiers DICOM du scanner (3) ET/OU
- Un appareil de télémétrie indépendant.

Les données provenant du dosimètre (6) sont les signaux échantillonnés de mesure de radiation détectées par la ou les fibres scintillantes (5). Ces données sont horodatées par le dosimètre (6) ou par le calculateur (10) commandant l'enregistrement dans la base de données (12).

Ces données concernent aussi la position horodatée de la table mobile (2), obtenue par un capteur de position ou par un accéléromètre intégré dans le boîtier du dosimètre (6) ou encore par un capteur de position intégré au scanner (3). La combinaison des informations de mesure d'irradiation et de position permet de calculer une information de doses détectées par la ou les fibres scintillantes (5) en fonction de la position sur l'axe longitudinal du patient (1).

Les données provenant du scanographe (3) sont le fichier DICOM comprenant l'image du patient colorisé en fonction des signaux fournis par les détecteurs du scanner, le rapport de dose structuré (type RDSR) et la mesure CTDI en microgray par exemple en fonction de la position transversale, pour une position longitudinale donnée.

Étapes (110 et 120) : prétraitement et exploitation des données

Ces données font l'objet de prétraitements par le serveur (10) :

- Prédécoupage des données mesurées par le dosimètre (6) pour ne garder que les séquences d'irradiation
- Prédécoupage des données DICOM du scanner (3) en différentes séquences pour faire la correspondance avec les mesures.

Le serveur (10) transmet à l'interface utilisateur des équipements (30) les données mesurées prétraitées pour affichage des courbes de mesure en temps réel.

L'ensemble des données récupérées et prétraitées est également transmis au serveur (20) qui est un serveur partagé entre plusieurs scanographes pour les traitements spécifiques.

Étape (200) : Mise en correspondance des données patient

Cette étape concerne la mise en correspondance des séquences de mesures avec les différentes acquisitions cliniques (contenu des fichiers DICOM correspondants)

Les données mesurées issues du dosimètre (6) doivent pouvoir être associées à un examen patient afin de pouvoir renseigner automatiquement la dose mesurée dans le dossier patient. Pour ce faire, on utilise une méthode algorithmique de fusion de modèles (« mergence algorithme) par exemple selon un traitement basé sur la triangulation de Delaunay. Ce traitement permet d'établir un lien entre les mesures réalisées par le dosimètre et les informations relatives à l'examen médical et au patient afin d'associer la dose mesurée au dossier patient :

- la correspondance entre les données d'irradiations issues du dosimètre et les images DICOM d'une part,
- la correspondance entre les images DICOM et les acquisitions référencées dans le rapport de dose DICOM SR d'autre part

L'établissement de ces correspondances permet de calculer les indicateurs de dose CTDI (Computed Tomography Dose Index) et l'estimation du facteur de risque des tissus (PDL) propres à chaque patient, et de les comparer aux valeurs indiquées par le scanner dans le rapport de dose et/ou le DICOM SR.

La difficulté de la fusion des données provient de la variété des examens et des protocoles cliniques associés. A titre d'exemple, l'établissement de la correspondance entre les irradiations et les images DICOM peut se faire en deux temps :

- La première étape consiste à déterminer quelles images et quelles séquences d'irradiation vont servir à l'établissement de la mergence (certaine images et séquences ne correspondant pas à un examen patient par exemple).
- La seconde consiste à faire correspondre les temps d'acquisitions (issus des images DICOM) et les temps d'irradiation (issus des mesures réalisées par le dosimètre) par une approche de minimisation de l'erreur semblable à la méthode des moindres carrés afin de faire correspondre chaque tir à une image et à une dose.

La mesure brute, associée à un tir ou à une série de tir (ou d'irradiation) réalisée par le scanner, peut ensuite être convertie en dose (en Gy ou en Sv) par l'application d'un facteur d'étalonnage.

En effet, les données d'irradiation issues du dosimètre sont des données brutes, non étalonnées. La fibre optique scintillante, lorsqu'elle est placée dans le champ d'irradiation (rayonnement direct ou diffusé), va capter et transformer l'énergie délivrée par le rayonnement du tube RX en photons lumineux selon un processus de scintillation. On mesure alors, par méthode de comptage des photons, une quantité de lumière qui est proportionnelle à la dose déposée dans la sonde.

Ainsi pour transformer la lumière mesurée en dose (dans l'unité choisie), notamment en Kerma dans l'air, Dose dans l'eau à la surface, dose dans l'eau en profondeur, dose efficace, etc..., une série de traitements est appliquée.

Étape (300) étalonnage et facteurs correctif à appliquer à la mesure de dose

Cette étape (300) consiste à appliquer un facteur d'étalonnage N, qui permet de relier les mesures du dosimètre (6) à une référence nationale. L'étalonnage du dosimètre est réalisé par un laboratoire d'étalonnage pour une ou plusieurs qualités de faisceau et dans une unité qui peut être le kerma dans l'air dans qualité de faisceau RQT9, (référence dans le domaine de la scanographie). Dans ce cas le facteur d'étalonnage correspondant est noté $N_{k(RQT9)}$.

Cette étape (300) comprend également la compensation de certaines sources de variabilité. Ainsi un premier traitement concerne l'amélioration de l'uniformité spatiale de réponse du détecteur et un deuxième traitement concerne la prise en compte de la dépendance en énergie du détecteur.

Étape (310) Compensation en uniformité de mesure

De part des caractéristiques, l'utilisation de fibre scintillante pour la détection de rayonnement permet une utilisation dans beaucoup de domaine jusqu'alors inaccessibles car les autres technologies présentent des caractéristiques non compatibles avec ces domaines. Par exemple, une chambre d'ionisation ou un détecteur à semi-conducteur ne pourra pas être utilisé comme dosimètre en imagerie en routine clinique car cela viendrait perturber les images, ces détecteurs n'étant pas radiotransparents, contrairement à une fibre plastique scintillante.

D'autre part, le faible encombrement d'une fibre scintillante la rend invisible et n'introduit aucune gêne pour le patient ce qui n'est pas le cas des autres technologies de mesure en temps réel.

Afin de satisfaire à la règlementation concernant les dosimètres dédiés au radiodiagnostic (IEC61674), la perte doit être inférieure à 3% tout le long de la partie active de détection. Ceci n'est pas le cas si aucune correction n'est apportée, même si le sondes sont confectionnées selon un protocole très strict. D'autre part, la réalisation de telles sondes, avec autant de contraintes au niveau de leur fabrication, n'est pas envisageable au niveau industriel car elles seraient bien trop onéreuses.

Les variations naturelles de réponse de la fibre scintillante le long de son axe doivent donc être compensées afin de garantir une uniformité de réponse sur l'ensemble de la « longueur assignée » de la sonde. La compensation se base sur l'application d'un modèle de compensation défini à partir de mesures réalisées pour chaque dosimètre lorsque celui-ci est installé sur un scanner. A titre d'exemple, ce modèle met en oeuvre les étapes suivantes :

Évaluation de la réponse moyenne notée R de la sonde sur l'ensemble de sa longueur grâce à une irradiation longitudinale progressive et continue de la fibre scintillante disposée sur la table du scanner telle que :

[Math.1]

$$\bar{R} = \frac{\int_0^{L_{sonde}}(I_1 + I_2)dz}{L_{sonde}}$$

Où $L_{sonde}$ correspond à la longueur de la fibre scintillante et $I_1$ et $I_2$ les mesures de lumières au niveau des deux extrémités de la sonde.

Évaluation de l'écart relatif entre les quantités de lumière mesurées en sortie des deux voies optiques $I_1$ et $I_2$. L'écart relatif, pour chaque échantillon i étant de la forme :

[Math.2]

$$E_r(i) = \frac{I_1(i) - I_2(i)}{I_1(i) + I_2(i)}$$

Évaluation de l'écart avec la réponse moyenne $\varepsilon(i) = \frac{I_1(i)+I_2(i)-\bar{R}}{\bar{R}}$ i correspond soit à une coordonnées temporelle soit à une coordonnées spatiale Évaluation de l'erreur de mesure en fonction de la position d'irradiation à partir de la régression linéaire quadratique suivante :

[Math.3]

$$\varepsilon = a \times E_r{}^2 + b \times E_r + c$$

Loi obtenue permettant de compenser l'erreur sur la mesure pour chaque position d'irradiation sur la fibre scintillante.

Étape (320) Compensation de la dépendance en énergie

Le dosimètre (6) fournit une mesure de dose pouvant présenter une dépendance en énergie suivant l'unité de dose en question : un scanner peut irradier avec plusieurs « qualités de faisceaux » différentes, et d'énergies moyenne différentes, selon l'examen clinique à réaliser. De ce fait, l'erreur de mesure peut atteindre +/- 30%. Un algorithme de correction de la dépendance en énergie a pour rôle de corriger cette situation.

La mise en application de cette correction nécessite donc la connaissance de la « qualité de faisceau » (i.e. de l'énergie moyenne du faisceau) pour chaque irradiation. Cette information n'étant pas fournie par le scanner, nous avons mis en place une série de mesures de calibration à chaque installation d'un dosimètre un scanner, afin de déterminer toutes les qualités de faisceaux disponibles pour chaque scanner et pour chaque type d'irradiation.

La mesure de la couche de demi-atténuation (CDA) est un exemple de méthode permettant de déterminer la qualité de faisceau.

Il est également possible de déterminer la « qualité de faisceau » correspondant à un examen par exemple par comparaison entre des dosimètres n'ayant pas le même matériau de détection (par exemple une chambre d'ionisation pour laquelle le milieu de détection est de l'air et notre dosimètre pour lequel le milieu de détection est du plastique).

La connaissance de la qualité de faisceau permet alors de définir un écart $\Delta E$ entre l'énergie moyenne du faisceau et l'énergie moyenne du faisceau utilisé lors de l'étalonnage du dosimètre.

La courbe de réponse du dosimètre (courbe de caractérisation dans différentes qualités de faisceau obtenue grâce à des mesures réalisées en laboratoire avec un appareil représentatif du dosimètre (6) et un dosimètre à chambre d'ionisation utilisé comme référence) permet ensuite d'appliquer la correction au facteur d'étalonnage.

Étape (400) Correction du facteur d'étalonnage

Ainsi, en considérant que l'appareil ai été étalonné en kerma dans l'air dans la qualité de faisceau RQT9, on obtient :

[Math.4]

$$Nk_{corrigé} = Nk_{RQT9} \times Nk^*$$

Où *Nk\** correspond au facteur de correction de la dépendance en énergie défini par l'écart entre la qualité de faisceau du scanner et la qualité de faisceau RQT9 utilisée pour l'étalonnage de l'appareil.

Après l'étape (400), on obtient une mesure de dose (ou de débit de dose) dans l'unité voulue (Gy ou Sv selon le « type » de dose : kerma dans l'air, dose dans l'eau, etc...)

Dans le domaine de la scanographie, les indicateurs dosimétriques devant être renseignés dans le dossier patient sont le CTDI (computed tomography dose index) et la DLP (Dose Length Product).

Afin de délivrer cette information il est ensuite nécessaire convertir la mesure de dose (i.e. transformer la courbe des débits de dose mesurés en un équivalent CTDI et DLP).

Étape (500) Transformation de la dose mesurée en indicateur de dose dédiée à la scanographie

Le calculateur (10) commande aussi un traitement pour calculer pour chaque patient, le CTDI et la DLP, pour chaque irradiation ou chaque série d'irradiations et pour l'ensemble d'un examen patient (i.e. somme de toutes les irradiations pour un même examen).

Ce traitement prend en compte, par application de facteurs correctifs, les cas où le patient n'est pas parfaitement positionné sur le lit, ce qui peut induire un biais non négligeable au niveau du facteur de conversion. Il consiste à appliquer un ou des facteur(s) de conversion des indices de dose (CTDI, PDL, dose efficace, dose à l'organe, etc.).

Le traitement permet de définir un CTDI(i) instantané pour chaque échantillon i correspondant à un point de mesure. L'intérêt de cette amélioration est de mieux prendre en compte notamment l'effet de modulation de dose des scanners et de mesurer plus précisément la dose déposée au niveau des organes à risque (i.e. les algorithmes de modulation de dose des scanner sont appliqués notamment pour réduire la dose lorsque le scanner passe au-dessus d'un organe sensible (poumons, cristallin, etc...).

Il est représenté de manière générale par :

[Math.5]

$$CTDI_{FMX}(i) = \frac{N_C \times Collimation}{1000 \ \times TableFeedPerRotation} \sum_{j=-\frac{P}{2}}^{\frac{P}{2}} \dot{K}a(i+j)$$

Où :

- P est le nombre d'échantillons pour un tour
- Ne est un facteur de conversion permettant de convertir la dose mesurée dans la fibre à la dose moyenne dans une tranche du volume irradié.
- $\dot{K}a(i)$ est l'échantillon i de la mesure instantanée de débit de kerma dans l'air (mGy.s$^{-1}$)
- N est le nombre total d'échantillons de l'acquisition
- Collimation est la collimation totale utilisée pour le tir (cm)

- TableFeedPerRotation correspond au déplacement de la table pendant la durée d'un tour (cm)
- Le facteur 1/1000 correspond à 1ms (s)

Et collimation/TableFeedPerRotation = 1 si l'acquisition n'est pas une hélice.

Les algorithmes donnant le CTDI moyen de l'irradiation, la DLP, et les indicateurs de dose cumulés par examen sont décrits dans le document cité plus haut.

Une fois ces indicateurs de dose définis, nous pouvons les comparer aux valeurs affichées par le scanner et évaluer l'écart

Ces écarts se calculent aussi bien pour les CTDI de l'examen que pour les CTDI des différents tirs. Cette formule est également valable pour définir les écarts de DLP.

Étape (600) Détermination de la position d'une irradiation et cartographie de dose

Cette étape concerne la corrélation entre les données corrigées, étalonnées et converties M(t) et les positions p à l'instant t => M(p)

Le fait d'avoir 2 canaux de mesure, à chaque extrémité de la sonde de détection permet de définir la position d'irradiation. Ceci est connu de la littérature et notamment décrit dans le brevet US5704890 (Page 9 - Détermination of location by pulse height).

Cette méthode étant jugée comme peu fiable avec une résolution spatiale peu satisfaisante, notamment compte tenu de la « pollution » du signal (= de la dose) mesuré due aux rayonnement diffusé et rétrodiffusé par le patient, nous avons utilisé cette technique uniquement afin de définir le sens de l'irradiation (mouvement du lit).

La combinaison de cette technique avec une analyse des images DICOM du scanner et/ou un quelconque élément permettant de mesurer une position à chaque instant t, permet de réaliser une conversion temps - espace c'est-à-dire convertir les temps d'irradiation mesurés en positions d'irradiation (pour chaque instant t pour lequel une dose est mesurée, on calcule une position i).

Ceci permet d'obtenir une valeur de dose et/ou de CTDI local pour chaque position d'irradiation et de cumuler toutes les irradiations d'un même examen le cas échéant.

Afin d'avoir un rendu visuel plus parlant et plus pertinent, une fois cette première conversion réalisée, on procède au passage dans le référentiel spatial du patient, c'est-à-dire qu'on fait correspondre à chaque position définie ci-dessus, un pixel d'une image du patient (cette image peut correspondre par exemple à un localiser encore appelée topogramme ou scout).

A titre d'exemple, un élément permettant de mesurer une position à chaque instant t peut être un accéléromètre. Il permet de déterminer la position de manière plus précise et peut venir enrichir la méthode utilisant uniquement une analyse des images DICOM du scanner. L'intérêt de son utilisation en complément de l'analyse des images DICOM est que si l'image n'est pas disponible il sera tout de même possible d'associer une position à chaque temps d'irradiation et donc procéder au cumul s'il y a plusieurs irradiations avec des recoupes.

Étape (700) : Cartographie de la dose

L'étape (600) décrite ci-dessus permet d'obtenir pour chaque position d'irradiation une valeur de dose/débit de dose et/ou CTDI(i). Le traitement réalise notamment la colorisation des images patient en fonction des mesures de dose calculées pour chaque position p.

Étape (800) : Exploitation sur le serveur (10)

Le centre de calcul (20) envoi les données traitées au serveur (10) qui sauvegarde les résultats localement et exploite les données pour afficher les résultats sur une interface graphique.

**Revendications**

1. Procédé de traitement des données relatives à un examen radiologique d'un patient au moyen d'un dispositif de détermination comportant l'acquisition des doses mesurées ($C_i$, $t_i$) à une pluralité d'instant $t_i$ et l'enregistrement de ces mesures de doses d'irradiation horodatées, et l'acquisition d'au moins un fichier numérique DICOM contenant des informations sur l'examen comportant les étapes suivantes :

   - Acquisition et enregistrement d'au moins un fichier numérique DICOM délivré par le tomographe (3) pendant ou après la réalisation d'une tomographie le procédé étant **caractérisé en ce qu'**il comporte en outre les étapes suivantes:
- Acquisition et enregistrement des mesures horodatées des doses détectées par une fibre scintillante (5) placée sur la table (2), et des déplacements horodatés de ladite table (2)
- interpolation desdites mesures ($C_i$, $t_i$) avec des données de l'image (DICOM) dans un espace interpolé commun et construction d'une table ($C_k$, $DICOM_k$) dans ledit espace interpolé
- détermination d'une table des niveaux de doses moyennes $T_z$ dans chaque tranche T en fonction des données ($DICOM_k$, $C_k$).

2. Procédé de traitement des données relatives à un examen radiologique d'un patient selon la revendication 1 **caractérisé en ce qu'**il comporte en outre une étape de détermination d'un facteur correctif pour la compensation de l'uniformité de mesure.

3. Procédé de traitement des données relatives à un examen radiologique d'un patient selon la revendication 1 **caractérisé en ce qu'**il comporte en outre une étape de détermination d'un facteur correctif pour la compensation des effets de dépendance en énergie.

4. Procédé de traitement des données relatives à un examen radiologique d'un patient selon la revendication précédente **caractérisé en ce que** ledit facteur correctif est déterminé en fonction de la différence entre le faisceau clinique et le faisceau utilisé pour l'étalonnage du dosimètre.

5. Procédé de traitement des données relatives à un examen radiologique d'un patient selon la revendication précédente **caractérisé en ce que** ledit facteur correctif est déterminé par un traitement consistant à définir dans un premier temps l'écart entre l'énergie moyenne du faisceau de rayons X clinique et l'énergie moyenne du faisceau de référence utilisé lors de l'étalonnage par une mesure de la couche de demi-atténuation (CDA) du faisceau clinique puis d'appliquer la correction du facteur d'étalonnage correspondant.

6. Procédé de traitement des données relatives à un examen radiologique d'un patient selon la revendication précédente **caractérisé en ce qu'**il comporte la succession d'étapes suivantes :

   - Etape (100) : acquisition et enregistrement des mesures brutes
   - Etape (110) : prétraitement des données
   - Etape (120) : exploitation des données
   - Etape (200) : Mise en correspondance des données patient
   - Etape (300) : correction des mesures brutes et étalonnage
   - Etape (400) : correction de l'étalonnage
   - Etape (500) : conversion en indicateurs de dose notamment CTDI DLP
   - Etape (600) : Conversion temps-espace
   - Etape (700) : cartographie des doses
   - Etape (800) : exploitation des données.

**Patentansprüche**

1. Verfahren zum Verarbeiten von Daten bezüglich einer Röntgenuntersuchung eines Patienten mittels einer Bestimmungsvorrichtung, das das Erfassen der gemessenen Dosen ($C_i$, $t_i$) zu einer Vielzahl von Zeitpunkten $t_i$ und das Aufzeichnen dieser Messungen von mit einem Zeitstempel versehenen Bestrahlungsdosen und das Erfassen mindestens einer digitalen DICOM-Datei, die Informationen über die Untersuchung enthält, aufweist, das die folgenden Schritte aufweist:

   - Erfassen und Speichern mindestens einer digitalen DICOM-Datei, die durch den Tomographen (3) während oder nach der Durchführung einer Tomographie ausgegeben wird, wobei das Verfahren **dadurch gekennzeichnet ist, dass** es ferner die folgenden Schritte aufweist:
   - Erfassen und Speichern der mit dem Zeitstempel versehenen Messungen der Dosen, die durch eine auf dem Tisch (2) platzierte Szintillationsfaser (5) erkannt werden, und der mit dem Zeitstempel versehenen Bewegungen des Tisches (2),
   - Interpolieren der Messungen ($C_i$, $t_i$) mit Bilddaten (DICOM) in einem gemeinsamen interpolierten Raum und Erstellen einer Tabelle ($C_k$, $DICOM_k$) in dem interpolierten Raum,
   - Bestimmen einer Tabelle der mittleren Dosenwerte $T_z$ in jedem Teilabschnitt T in Abhängigkeit von den Daten

(DICOM$_k$, C$_k$).

**2.** Verfahren zum Verarbeiten von Daten bezüglich einer Röntgenuntersuchung eines Patienten nach Anspruch 1, **dadurch gekennzeichnet, dass** es ferner einen Schritt zum Bestimmen eines Korrekturfaktors für die Kompensation der Messuniformität aufweist.

**3.** Verfahren zum Verarbeiten von Daten bezüglich einer Röntgenuntersuchung eines Patienten nach Anspruch 1, **dadurch gekennzeichnet, dass** es ferner einen Schritt zum Bestimmen eines Korrekturfaktors für die Kompensation von Wirkungen einer Energieabhängigkeit aufweist.

**4.** Verfahren zum Verarbeiten von Daten bezüglich einer Röntgenuntersuchung eines Patienten nach dem vorstehenden Anspruch, **dadurch gekennzeichnet, dass** der Korrekturfaktor in Abhängigkeit von der Differenz zwischen dem klinischen Strahl und dem für die Kalibrierung des Intensimeters verwendeten Strahl bestimmt wird.

**5.** Verfahren zum Verarbeiten von Daten bezüglich einer Röntgenuntersuchung eines Patienten nach dem vorstehenden Anspruch, **dadurch gekennzeichnet, dass** der Korrekturfaktor durch eine Verarbeitung bestimmt wird, die darin besteht, zunächst die Abweichung zwischen der mittleren Energie des klinischen Röntgenstrahls und der mittleren Energie des während der Kalibrierung verwendeten Referenzstrahls durch eine Messung der Halbwertschicht (CDA) des klinischen Strahls zu definieren und dann die Korrektur des entsprechenden Kalibrierungsfaktors anzuwenden.

**6.** Verfahren zum Verarbeiten von Daten bezüglich einer Röntgenuntersuchung eines Patienten nach dem vorstehenden Anspruch, **dadurch gekennzeichnet, dass** es die Abfolge der folgenden Schritte aufweist:

- Schritt (100): Erfassen und Speichern von Rohmessungen
- Schritt (110): Vorverarbeiten der Daten
- Schritt (120): Auswerten der Daten
- Schritt (200): Zuordnen der Patientendaten
- Schritt (300): Korrigieren der Rohmessungen und der Kalibrierung
- Schritt (400): Korrigieren der Kalibrierung
- Schritt (500): Umrechnen in Dosisbezeichner, insbesondere CTDI DLP
- Schritt (600): Raum-Zeit-Umwandeln
- Schritt (700): Kartografieren der Dosen
- Schritt (800): Auswerten der Daten.

**Claims**

**1.** Method for processing data relating to a radiological examination of a patient by means of a determining device, the method comprising acquiring doses (C$_i$, t$_i$) measured at a plurality of times t$_i$, storing these time-stamped measurements of radiation doses, and acquiring at least one DICOM digital file containing information on the examination, comprising the following steps:

- acquiring and storing at least one DICOM digital file delivered by the tomograph (3) during or after the performance of tomography, the method being **characterized in that** it further comprises the following steps:
- acquiring and storing time-stamped dose measurements detected via a scintillating fiber (5) placed on the table (2), and time-stamped movements of said table (2)
- interpolating said measurements (C$_i$, t$_i$) with data from the image (DICOM) in a common interpolated space and constructing a table (C$_k$, DICOM$_k$) in said interpolated space
- determining a table of average dose levels T$_z$ in each slice T according to the data (DICOM$_k$, C$_k$).

**2.** Method for processing data relating to a radiological examination of a patient according to claim 1, **characterized in that** it further comprises a step of determining a corrective factor for compensation for measurement consistency.

**3.** Method for processing data relating to a radiological examination of a patient according to claim 1, **characterized in that** it further comprises a step of determining a corrective factor for compensation for energy dependence effects.

**4.** Method for processing data relating to a radiological examination of a patient according to the preceding claim, **characterized in that** said corrective factor is determined according to the difference between the clinical beam and the beam used for calibrating the dosimeter.

5. Method for processing data relating to a radiological examination of a patient according to the preceding claim, **characterized in that** said corrective factor is determined by processing consisting of firstly defining the difference between the average energy of the clinical X-ray beam and the average energy of the reference beam used during calibration by measuring the half-value layer (HVL) of the clinical beam, then applying the correction of the corresponding calibration factor.

6. Method for processing data relating to a radiological examination of a patient according to the preceding claim, **characterized in that** it comprises the following series of steps:

   - Step (100): acquiring and storing raw measurements
   - Step (110): preprocessing the data
   - Step (120): using the data
   - Step (200): mapping patient data
   - Step (300): correcting raw measurements and calibrating
   - Step (400): correcting the calibration
   - Step (500): converting into dose indicators, in particular CTDI DLP
   - Step (600): time-space conversion
   - Step (700): dose mapping
   - Step (800): using the data.

[Fig. 1]

[Fig. 2]

[Fig. 3]

| Etape 100 : Acquisition par le serveur (10) des données brutes | | |
|---|---|---|
| Données brutes du dosimètre (6) | fichiers DICOM du scanner (3) | Données spatiales (15) |

| Etape (110) : le serveur (10) prétraite les données | |
|---|---|
| Prédécoupage des données du dosimètre (3) | Prédécoupage des données DICOM du scanner (3) |

| Etape (120) : le serveur DICOM (10) renvoie | |
|---|---|
| Affichage des courbes de mesure en temps réel | Transfert des données au serveur (20) |

| Etape (200) Le serveur (20) assemble les données |
|---|
| Mise en correspondance des séquences de mesures avec les différentes acquisitions cliniques |

| Etape (300) : étalonnage et correction des mesures brutes | |
|---|---|
| (310) correction de l'uniformité de mesure. | (320) correction de la dépendance en énergie |

| Etape (400) : calibration des données par le serveur (20) |
|---|
| Correction du facteur d'étalonnage (unité de référence ex : Kerma dans l'air, dose dans l'eau). |

| Etape (500) : conversion des données par le serveur (20) |
|---|
| Application des facteurs de conversion des indices de dose (CTDI, PDL, dose efficace, dose à l'organe, etc...). |

| Etape (600) conversion temps <=> espace |
|---|
| Corrélation entre les données corrigées, étalonnées et converties M(t) et les position p à l'instant t => M(p) |

| Etape (700) : cartographies de dose |
|---|
| Colorisation des images patient en fonction des mesures de dose calculées pour chaque position p |

| Etape (800) : exploitation sur le serveur (10) | |
|---|---|
| Sauvegarde les données | 4. Affiche les résultats sur l'interface utilisateur |

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- US 7627079 B2
- KR 20150061520 A
- US 8189740 B
- FR 3053799
- WO 2012129661 A1
- US 8714818 B
- WO 2019064652 A
- US 2006018435 A
- US 2006027756 A
- US 5704890 A